# EUROPÄISCHE PATENTSCHRIFT

(11) **EP 2 167 977 B1**
(45) Veröffentlichungstag und Bekanntmachung des Hinweises auf die Patenterteilung: **31.10.2012**
(21) Anmeldenummer: 08772578.4
(22) Anmeldetag: 04.07.2008
(51) Int. Cl.: G01N 33/86

(54) **VERFAHREN ZUR BESTIMMUNG THROMBOGENER MIKROPARTIKEL**
METHOD FOR DETERMINING THROMBOGENIC MICROPARTICLES
PROCÉDÉ D'IDENTIFICATION DE MICROPARTICULES THROMBOGÈNES

(30) Priorität: 06.07.2007 AT 10462007
(43) Veröffentlichungstag der Anmeldung: 31.03.2010
(73) Patentinhaber: Technoclone Gesellschaft M.B.H., 1235 Wien (AT)
(72) Erfinder: BINDER, Bernd, A-1010 Wien (AT); VETR, Helga, A-2340 Mödling (AT); ISOBE, Hirotaka, 862-0935 Japan (JP); BINDER, Christoph, A-1010 Wien (AT)
(74) Vertreter: Patentanwälte Barger, Piso & Partner
(86) Internationale Anmeldenummer: PCT/AT2008/000244
(87) Internationale Veröffentlichungsnummer: WO 2009/006659

(56) Entgegenhaltungen:
- WO-A-2006/089324
- KAUFMANN VIKTORIA ET AL: "Microparticles area major determinant of thrombin generation measured by Technothrombin (R) TGA." BLOOD, Bd. 108, Nr. 11, Part 1, November 2006 (2006-11), Seite 423A, XP002501565 & 48TH ANNUAL MEETING OF THE AMERICAN-SOCIETY-OF-HEMATOLOGY; ORLANDO, FL, USA; DECEMBER 09 -12, 2006 ISSN: 0006-4971
- JY W ET AL: "Measuring circulating cell-derived microparticles." JOURNAL OF THROMBOSIS AND HAEMOSTASIS : JTH OCT 2004, Bd. 2, Nr. 10, Oktober 2004 (2004-10), Seiten 1842-1851, XP002501566 ISSN: 1538-7933
- VETR HELGA ET AL: "Evaluation of a method for removal and determination of thrombogenic microparticles" BLOOD, Bd. 110, Nr. 11, Part 1, November 2007 (2007-11), Seite 1061A, XP009107804 & 49TH ANNUAL MEETING OF THE AMERICAN-SOCIETY-OF-HEMATOLOGY; ATLANTA, GA, USA; DECEMBER 08 -11, 2007 ISSN: 0006-4971

## Beschreibung

Die Erfindung betrifft ein Verfahren zur Bestimmung von Zahl und funktioneller Aktivität thrombogener Mikropartikel in Plasma entsprechend dem einleitenden Teil des Anspruches 1.

### Hintergrund der Erfindung

### Pathologische Bedeutung von Mikropartikeln

Seit einigen Jahren gibt es immer mehr Hinweise darauf, dass sog. "zelluläre Mikropartikel" eine wesentliche Rolle bei der Entstehung von Thrombosen und anderen Krankheitsbildern wie Entzündung und/oder Infektionen spielen (Übersichtsarbeiten dazu sind in der Literaturliste angegeben).

Mikropartikel (MP) sind kleine, von der Zellmembran stammende Vesikel, die sowohl von Endothelzellen als auch von allen im Blut zirkulierenden Zelltypen unter bestimmten Bedingungen in den Blutstrom abgegeben werden können. MP sind maximal 1 µm groß und ihre Membranoberfläche enthält Phospholipide, Oberflächenproteine aus der Ursprungszelle und Proteine, wie z.B. "tissue factor", die zusammen mit den Phospholipiden eine gerinnungsfördernde Oberfläche bilden. Zellen können durch verschiedenste Faktoren zur Abgabe von MP stimuliert werden. Dazu gehören Zell-Lyse durch Komplement-Aktivierung ebenso wie oxidativer Stress oder hohe Scherkräfte. Aufgrund ihres unterschiedlichen zellulären Ursprungs und der unterschiedlichen "Trigger", durch die sie generiert werden, sind Mikropartikel bezüglich ihrer Größe und Zusammensetzung der Oberfläche sehr heterogen.

### Derzeitige Methoden zur Bestimmung von Mikropartikeln

Die aktuellen "state-of the art" Methoden für die Bestimmung von Mikropartikeln verwenden entweder durchfluss-zytometrische Analysen (FACS) oder ELISA-Bestimmungen. Die Anzahl der Mikropartikel wird durchfluss-zytometrisch bestimmt, wobei alle Ereignisse zwischen 0,1 µm und 1.0µm gezählt werden. Für die Kalibrierung stehen entsprechende Partikelstandards zur Verfügung⁷. Alternativ dazu können spezifische Oberflächenstrukturen für die Bestimmung sowohl im Durchfluss-Zytometer als auch in immunologischen Verfahren (ELISA) verwendet werden. Hier wird in der Regel die Eigenschaft ausgenutzt, dass negativ geladene Phospholipide, wie sie an der Oberfläche von MP vorhanden sind, spezifisch an Annexin V binden. Alternativ oder ergänzend zur Annexin V Bindung können Antikörper, die gegen Oberflächenproteine auf MP gerichtet sind, zur Bestimmung des zellulären Ursprungs von Mikropartikeln verwendet werden. Diese Oberflächenmarker sind typisch für die entsprechenden Herkunftszellen, wie Endothelzellen, Neutrophile, Monozyten oder Erythroyzyten. Prinzipiell wird in der WO2006/089324 A1 geoffenbart, dass Mikropartikel nicht nur die Thrombingenerierung in Plasma beeinflussen, sondern auch, dass der Zusatz von Mikropartikel zu mikropartikelfreiem Plasma die Thrombingenerierung dosisabhängig steigert. In WO2006/089324 A1 werden jedoch Gewebefaktor-freie Phospholipidmizellen als Trigger der Thrombingenerierung verwendet, sodass insbesondere der Gewebefaktor-Gehalt der Mikropartikel von Einfluss auf die Thrombingenerierung ist und nicht deren Phospholipidgehalt, da diese als Trigger zugestzt werden. Weiters wird in WO2006/089324 A1 thrombozytenfreies und nicht mikropartikelfreies Plasma als Basis der Thrombingenerierung verwendet, zu der die Thrombingenerierung in Mikropartikelhältigem Plasma in Relation gesetzt wird.

### Nachteile der existierenden Methoden zur Mikropartikelbestimmung

Alle oben beschriebenen Verfahren analysieren Strukturen bzw. Moleküle auf der Oberfläche der Mikropartikel. Sie ermöglichen aber keine Aussage über die Funktionalität der Mikropartikel. Außerdem sind diese Verfahren auf die Untersuchung von Subpopulationen der MP beschränkt. Darüber hinaus wurde gezeigt, dass auch der prinzipiell zelltyp-unabhängige Marker Annexin V nicht universell ist, da nicht alle MP Annexin V positiv sind.

Allen Methoden gemeinsam ist die Einschränkung durch die Probenvorbereitung. Üblicherweise werden Mikropartikel durch Zentrifugation gewonnen. Es wurde jedoch gezeigt, dass mit Ultrazentrifugation nicht 100% der Mikropartikel aus Blutplasma gewonnen werden können, da einige MP dieselbe Dichte wie Plasma besitzen. Darüber hinaus ist die Gewinnung von MP durch Zentrifugation nur schwer standardisierbar und in den meisten Fällen nicht quantitativ. Die Alternative der Dichtegradienten-Zentrifugation ist nur für Forschungslabors geeignet und zeit- und kostenintensiv.

In der Beurteilung der Durchführbarkeit der genannten Methoden muss noch berücksichtigt werden, dass die Durchfluss-Zytometrie sehr spezielle und teure Ausstattung mit Geräten erfordert und in der Regel in einem klinischen Routinelabor nicht zur Verfügung steht.

### Ziel der vorliegenden Erfindung

Wie bereits beschrieben, hat keine der zur Zeit verfügbaren Methoden das Potential, sich als klinische Routine-Methode zu etablieren. Alle Verfahren haben den Nachteil, dass sie entweder teure Geräte benötigen, oder arbeitsintensiv sind, oder nur einen Teil der thrombogenen Mikropartikel erfassen. Darüber hinaus ist die Standardisierung dieser Methoden, die eine Voraussetzung für klinische Routinemethoden ist, nur schwer zu erreichen.

Aus all diesen Gründen wäre es wünschenswert, eine einfache, standardisierbare Methode zu haben, mit der sowohl Zahl als auch Funktion zirkulierender Mikropartikel zuverlässig bestimmt werden kann.

Das Ziel der vorliegenden Erfindung ist es daher, eine Methode anzubieten, mit der die schnelle, zuverlässige und reproduzierbare Bestimmung thrombogener Mikropartikel erreicht werden kann.

### Zusammenfassung der Erfindung

Das Ziel der vorliegenden Erfindung ist es, die Methode der Thrombingenerierung so zu modifizieren, dass damit die Zahl und die funktionelle Aktivität thrombogener Mikropartikel in Plasma bestimmt werden können. Dies wird erreicht durch Bestimmung der Thrombingenerierung in PPP (plättchenarmes Plasma) und MPFP (mikropartikelfreies Plasma), die aus derselben Probe gewonnen werden. Berechnet man die Differenz der Thrombingenerierung in diesen zwei Proben und vergleicht sie mit der Thrombingenerierung eines Mikropartikel-Standards, so erhält man eine Aussage sowohl über Anzahl als auch Aktivität der in der ursprünglichen Probe enthaltenen Mikropartikel. Dabei wird gemäß der Erfindung MPFP durch Filtration gewonnen.

Öffenbart ist auch ein Test-Kit, der alle nötigen Hilfsmittel und Verfahrensbeschreibungen enthält, die sowohl die Probenvorbereitung betreffen, als auch die Reagenzien zur Messung der Thrombingenerierung. Außerdem sind Standards und Kontrollen zur Quantifizierung der Zahl der Mikropartikel enthalten.

In einer weiteren Ausführungsform ist die Option für die automatisierte Probenanalyse beschrieben.

Diese Option beeinhaltet die Gewinnung der filtrierten Proben in Gefäßen, die mit einem automatisierten Gerinnungs-Analyzer des Typs CEVERON® ALPHA mit "TGA Modul" kompatibel sind.

### Kurze Beschreibung der Zeichnungsfiguren:

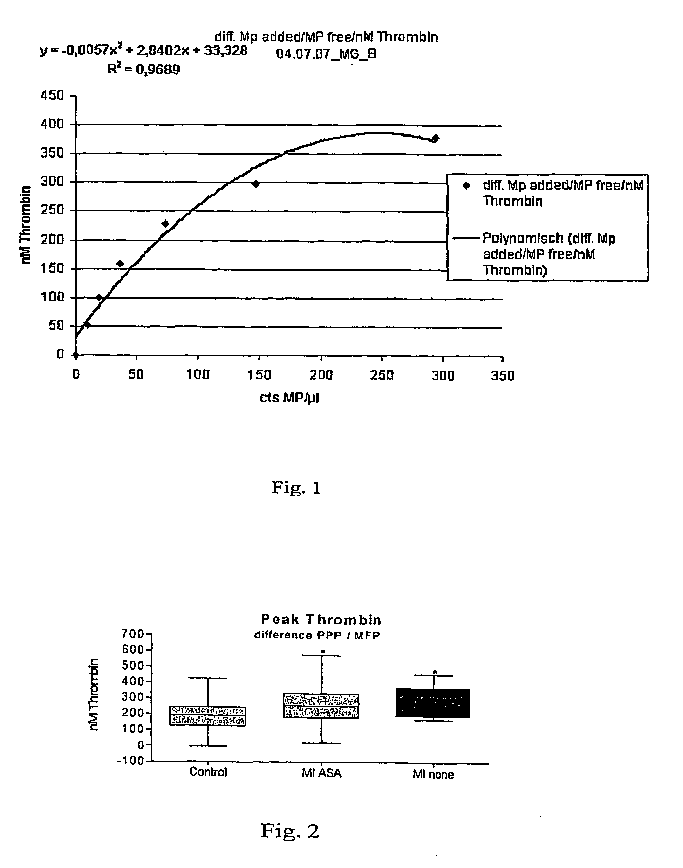

Fig. 1 stellt eine Eichkurve dar, in der die Zahl der MP(x 100) gegen nM Thrombin (peak thrombin) aufgetragen ist. Auf der Abszisse ist die Zahl der Mikropartikel pro µl (cts MP/µl) und auf der Ordinate nM peak Thrombin als Differenz der Thrombingenerierung zwischen Mikropartikel (MP) hältigem (MP added) und MP freiem (MP free) plasma dargestellt. Ebenso ist die Kurvengleichung angegeben
Fig. 2 zeigt die Unterschiede im "peak thrombin" zwischen PPP und MPFP. Aus diesen in Fig. 2 gezeigten Unterschieden zwischen PPP und MPFP kann die Anzahl an Mikropartikeln berechnet werden. Control (Kontroll-Personen), MI ASA (Patienten mit Herzinfarkt und Behandlung mit Aspirin), MI none (Patienten mit Herzinfarkt)

### Beispiele

### Bestimmung der Thrombin generierenden Aktivität (TGA) von Mikropartikeln Standards

Werden unterschiedliche Konzentrationen von isolierten Mikropartikeln zu standardisiertem mikropartikel-freiem Plasma gemischt, und danach die Thrombingenerierung bestimmt, so erhält man eine Standard-Kurve wie in Fig. 1 gezeigt.

### Probenvorbereitung und Thrombingenerierung

Aus antikoaguliertem Blut wird plättchenarmes Plasma (PPP) durch Zentrifugation entsprechend DIN 58905 (15 min bei mindestens 2500g) gewonnen. Mikropartikelfreies Plasma (MPFP) wird aus PPP durch Filtration gewonnen. Dazu wird eine Filtrationsmembran mit geringer Protein-Bindungskapazität und einer Poren-Größe von 0,2 µm verwendet (z.B. PALL, BioInert®). Die bevorzugte Filtrationseinheit ist eine AcroPrep® 96 well Filterplatte (PALL 5042) mit einer Bio-Inert® Membran (hydrophiles Nylon). Andere Filterplatten mit ähnlichen Eigenschaften können verwendet werden. Als Alternative kann auch Zentrifugation zur Gewinnung des MPFP verwendet werden.

Für jede Probe wird die Thrombingenerierung in PPP und MPFP bestimmt. Der Unterschied in der Thrombingenerierung von PPP und MPFP wird berechnet und daraus die Zahl der Mikropartikel in der Probe bestimmt. Als Parameter aus der Thrombingenerierungs-Kurve kann entweder "peak thrombin" oder die Fläche unter der Kurve verwendet werden. Durch Vergleich mit der Standardkurve (Fig. 1) kann die Konzentration der thrombogenen Mikropartikel in der Probe bestimmt werden. In Fig. 2 sind Mittelwerte für Thrombingenerierung in gesunden Kontrollen und in Proben aus verschiedenen Patientengruppen gezeigt, sowohl für PPP als auch für MPFP.

### Berechnung der Zahl der thrombogenen Mikropartikel

Die Unterschiede in der Thrombin generierenden Aktivität (z.B. im "peak thrombin") zwischen plättchenarmen Plasma (PPP) und mikropartikel-freiem Plasma (MPFP) werden berechnet und der Wert für die Zahl der Mikropartikel wird von der Standardkurve abgelesen.

| TGA in PPP nM thrombin | TGA in MPFP nM thrombin | Unterschied nM thrombin | Mikropartikel/µl |
|---|---|---|---|
| 125 | 27 | 98 | 2.200 |
| 387 | 75 | 312 | 15.000 |
| 258 | 43 | 215 | 7.500 |
| 175 | 44 | 131 | 4.000 |

## Patentansprüche

1. Verfahren zur Bestimmung von Zahl und funktioneller Aktivität thrombogener Mikropartikel in Plasma durch Bestimmung der Thrombingenerierung in PPP ("Platelet poor plasma") und MPFP (microparticle free plasma), wobei PPP und MPFP aus derselben Probe gewonnen werden, die Differenz dieser beiden Werte berechnet wird und auf die thrombogene Aktivität eines Mikropartikel Standards bezogen wird, **dadurch gekennzeichnet, dass** MPFP durch Filtration gewonnen wird.

## Claims

1. Method for determining the number and functional activity of thrombogenic microparticles in plasma by determining the thrombin generation in PPP (platelet poor plasma) and MPFP (microparticle free plasma), wherein PPP and MPFP are generated from the same sample, the difference between these two values is calculated and related to the thrombogenic activity of a microparticle standard, **characterised in that** MPFP is generated by filtration.

## Revendications

1. Procédé de détermination du nombre et de l'activité fonctionnelle de microparticules thrombogéniques dans le plasma par détermination de la génération de thrombine dans le plasma pauvre en plaquettes (PPP) et dans le plasma exempt de microparticule (MPFP, microparticle free plasma), dans lequel on recueille le PPP et le MPFP du même échantillon, on calcule la différence entre ces deux valeurs et on la rapporte à l'activité thrombogénique d'un étalon de microparticules, **caractérisé en ce que** le MPFP est recueilli par filtration.
